# EUROPEAN PATENT APPLICATION

(11) **EP 4 613 857 A1**
(43) Date of publication of application: **10.09.2025**
(21) Application number: 23895685.8
(22) Date of filing: 21.02.2023
(51) Int. Cl.: C12N 9/78, C12N 15/55, C12P 13/02, C12P 7/40, C12P 41/00

(54) **AMIDASE MUTANT AND USE THEREOF**

(30) Priority: 02.12.2022 CN 202211539071
(71) Applicant: Asymchem Life Science (Tianjin) Co., Ltd, Tianjin 300457 (CN)
(72) Inventor: HONG, Hao, Morrisville, North Carolina 27560 (US); JAMES, Gage, Morrisville, North Carolina 27560 (US); ZHANG, Na, Tianjin 300457 (CN); JIAO, Xuecheng, Tianjin 300457 (CN); MA, Yulei, Tianjin 300457 (CN); MU, Huiyan, Tianjin 300457 (CN); DAI, Hongqian, Tianjin 300457 (CN); LI, Hongxuan, Tianjin 300457 (CN); MA, Tianjiao, Tianjin 300457 (CN); ZUO, Jian, Tianjin 300457 (CN); WU, Jing, Tianjin 300457 (CN); DU, Xin, Tianjin 300457 (CN); TANG, Jingwei, Tianjin 300457 (CN); LU, Guosheng, Tianjin 300457 (CN)
(74) Representative: CAPRI
(86) International application number: PCT/CN2023/077496
(87) International publication number: WO 2024/113509

(57) **Abstract**

The present application provides an amidase mutant and use thereof. The amidase mutant comprises: (a) a protein having an amino acid sequence as shown in SEQ ID NO: 1; or (b) a protein having amidase functions after amino acid mutation at least one of the following sites of the amino acid sequence as shown in (a): L81, E62, M311, N180, P121, R58, E32, T230, N82, N190, D192, D217, H219, K77, M78, P79, F80, F61, D185, D59, L182, T183, P184, I227, C228, G229, A231 or V232; or (c) a protein having 80% or more identity with the amino acid sequence defined in any one of (a) and (b) and having amidase functions. The present application can solve the problems of low stereoselectivity and catalytic activity of amidases in the prior art, and is suitable for the field of enzyme catalysis.

## Description

The present application is based on and claims priority of a Chinese Patent Application No. 202211539071.X, filed on 02 December 2022, and the disclosure of which is incorporated again herein by reference in its entirety.

### Technical Field

The present application relates to the field of enzyme catalysis, and in particular to an amidase mutant and use thereof.

### Background

Chiral carboxylic acids and amide derivatives are key intermediates for synthesizing a large number of chiral bioactive molecules, and are widely used in the fields of fine chemical engineering, medicine, agricultural chemicals, functional materials, etc. The chemical synthesis of chiral carboxylic acids and amide derivatives has limitations such as a low total yield of product, poor selectivity and high costs of synthesis process, and is therefore not suitable for industrial scale production.

Amidase (EC 3.5.1.X) is an important class of hydrolases that catalyze the hydrolysis of amide compounds to produce carboxylic acids and ammonia. The amidases have a broad substrate spectrum which covers aliphatic, aromatic, heterocyclic compounds, etc. The amidases generally also have good stereoselectivity. S-type amidases are the most common, but R-type amidases have also been discovered in recent years. These characteristics of amidases make them have incomparable advantages in the kinetic resolution of racemic amides to prepare chiral carboxylic acids and chiral amide derivatives, and thus the amidases have attracted increasing attention from researchers. A series of new processes for the biosynthesis of chiral carboxylic acids and/or amide derivatives by using an amidase as a catalyst have been developed, and the chiral carboxylic acids and/or amide derivatives include important pharmaceutical intermediates such as S-2,2-dimethylcyclopropanecarboxamide, 1-cyanocyclohexylacetic acid, R-3,3,3-trifluoro-2-hydroxy-2-methylpropionic acid, and 2-chloronicotinic acid.

Wild-type amidases (obtained from nature) have certain limitations, and their deficiencies in catalytic activity, stereoselectivity and stability restrict the application in industrial production. However, with the continuous development of protein engineering and the deeper understanding of structure and functions of amidases, the application of enzyme evolution technology makes it possible to alter the catalytic properties of enzymes. The patent CN 107937376 B discloses a Pantoea-derived amidase for synthesizing 2-chloronicotinic acid, the activity of the enzyme is increased by 2-3 times through enzyme evolution, and the tolerance to the substrate chloronicotinamide exceeds 200 mM. In addition, Tang, X. L., et al. directionally evolved amidases from Pantoea sp., so that the catalytic activity of the amidases was improved, the activity of single mutant G175A and double mutant G175A/A305T was improved by 3.2-fold and 3.7-fold, respectively, and the Kcat/Km value was improved to 3.1-fold and 10.0-fold of wild-type amidases (Appl Environ Microbiol 2019 85(5): e02471-18). Therefore, it is very necessary to change the catalytic properties of wild-type amidases through enzyme evolution and promote enzyme catalysis to adapt to the needs of corresponding industrial production.

### Summary

The main object of the present application is to provide an amidase mutant and use thereof, so as to solve the problems of low stereoselectivity and catalytic activity of wild-type amidases in the prior art.

In order to achieve the described object, according to a first aspect of the present application, provided is an amidase mutant, comprising: (a) a protein having an amino acid sequence as shown in SEQ ID NO: 1; or (b) a protein having amidase functions after amino acid mutation at least one of the following sites of the amino acid sequence as shown in (a): L81, E62, M311, N180, P121, R58, E32, T230, N82, N190, D192, D217, H219, K77, M78, P79, F80, F61, D185, D59, L182, T183, P184, I227, C228, G229, A231 or V232; or (c) a protein having 80% or more identity with the amino acid sequence defined in any one of (a) and (b) and having amidase functions.

Further, the amino acid mutations of the described (b) are each independently selected from L81A or L81C or L81V; P79Q; F80G; E62Q or E62K or E62M or E62T or E62A or E62R or E62C or E62V or E62P or E62H or E62N or E62S; L76V; K77T; D185H; R58M or R58K or R58L or R58H or R58A; I227L or I227V or I227A or I227W or I227E or I227S or I227M or I227D or I227C or I227Y; T230C or T230V or T230A or T230L; N180V or N180D or N180K; P121H or P121K or P121N or P121A or P121R or P121E or P121Y or P121S; M311C or M311R or M311H or M311S; V232I; R58L or R58M or R58K or R58Q or R58P or R58A or R58V or R58F; E32D or E32H or E32M or E32Q or E32A or E32C or E32V or E32F or E32I or E32P or E32T; wherein the letter before the number represents the original amino acid, and the letter after the number represents the amino acid after mutation; and preferably, (c) is a protein has 85% or more, preferably 90% or more, more preferably 95% or more, and even more preferably 99% or more identity with the amino acid sequence defined in (a) or (b) and has amidase functions.

Further, the mutation of the described amidase mutant includes any one of the following amino acid mutations: L81A; L81C; L81V; P79Q; F80G; E62Q; E62K; E62M; E62T; L76V; K77T; D185H; R58M; I227L; I227V; T230C; L81A + E62T; L81A + E62K; L81A + E62M; L81A + I227V; L81A + T230C; L81A+A231R; L81A + L76V; L81A + I227V; L81A + N180V; L81A+ N180D; L81A + N180K; L81A + P121H; L81A + P121K; L81A + P121N; L81A + P121A; L81A + M311C; L81A + M311R; L81A + M311H; L81A + M311S; L81A + E62T + P121A; L81A + E62T + P121H; L81A + E62T + P121N; L81A + E62T + P121R; L81A + E62T + I227A; L81A + E62T + M311C; L81A + E62T + M311R; L81A + E62T + M311H; L81A + E62T + M311S; L81A + E62T + T230C; L81A + E62T + T230V; L81A + E62T + V232I; L81A + E62T + R58L; L81A + E62T + R58M; L81A + E62T + R58K; L81A + E62T + R58Q; L81A + E62T + E32D; L81A + E62T + E32H; L81A + E62T + E32M; L81A + E62T + M311C + E32Q; L81A + E62T + M311C + E32M; L81A + E62T + M311C + E32D; L81A + E62T + M311C + E32A; L81A + E62T + M311C + E32C; L81A + E62T + M311C + E32V; L81A + E62T + M311C + P121E; L81A + E62T + M311C + P121A; L81A + E62T + M311C + P121H; L81A + E62T + M311C + P121R; L81A + E62T + M311C + P121N; L81A + E62R + M311C; L81A + E62A + M311C; L81A + E62C + M311C; L81A + E62V + M311C; L81A + E62P + M311C; L81A + E62M + M311C; L81A + E62T + M311C + R58K; L81A + E62T + M311C + R58L; L81A + E62T + M311C + R58H; L81A + E62T + M311C + R58A; L81A + E62T + M311C + I227A; L81A + E62T + M311C + I227L; L81A + E62T + M311C + I227W; L81A + E62T + M311C + I227E; L81A + E62T + M311C + I227S; L81A + E62T + M311C + T230V; L81A + E62T + M311C + T230C; L81A + E62T + M311C + T230A; L81A + E62T + M311C + T230L; L81A + E62A + M311C + T230C; L81A + E62H + M311C + T230C; L81A + E62N + M311C + T230C; L81A + E62R + M311C + T230C; L81A + E62S + M311C + T230C; L81A + E62Q + M311C + T230C; L81A + E62T + M311C + T230C + R58K; L81A + E62T + M311C + T230C + R58P; L81A + E62T + M311C + T230C + R58A; L81A + E62T + M311C + T230C + R58V; L81A + E62T + M311C + T230C + R58F; L81A + E62T + M311C + T230C + R58M; L81A + E62T + M311C + T230C + I227V; L81A + E62T + M311C + T230C + I227M; L81A + E62T + M311C + T230C + I227A; L81A + E62T + M311C + T230C + I227D; L81A + E62T + M311C + T230C + I227C; L81A + E62T + M311C + T230C + I227Y; L81A + E62T + M311C + T230C + P121A; L81A + E62T + M311C + T230C + P121R; L81A + E62T + M311C + T230C + P121E; L81A + E62T + M311C + T230C + P121H; L81A + E62T + M311C + T230C + P121N; L81A + E62T + M311C + T230C + P121Y; L81A + E62A + M311C + T230C + E32F; L81A + E62A + M311C + T230C + E32I; L81A + E62A + M311C + T230C + E32P; L81A + E62A + M311C + T230C + E32T; L81A + E62A + M311C + T230C + I227V; L81A + E62A + M311C + T230C + I227L; L81A + E62A + M311C + T230C + I227A; L81A + E62A + M311C + T230C + T230V; L81A + E62A + M311C + T230C + P121A; L81A + E62A + M311C + T230C + P121R; L81A + E62A + M311C + T230C + P121S.

In order to achieve the described object, according to a second aspect of the present application, provided is a DNA molecule encoding the described amidase mutant.

In order to achieve the described object, according to a third aspect of the present application, provided is a recombinant vector linked with the described DNA molecule.

In order to achieve the described object, according to a fourth aspect of the present application, provided is a host cell transformed with the described recombinant vector.

In order to achieve the described object, according to a fifth aspect of the present application, provided is a method for preparing a chiral carboxylic acid and an amide derivative. The preparation method comprises using the described amidase mutant to catalyze a hydrolysis and resolution reaction of an amide substrate as represented by formula I, 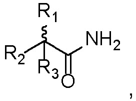 so as to obtain a chiral carboxylic acid and an amide derivative, wherein in formula I, R1 is selected from an aryl, a heteroaryl, an alkyl, an alkylene, a cycloalkyl, a hydroxyl, an amino or H, and is preferably the hydroxyl when selected from the hydroxyl, the amino or H; R2 is selected from an aryl, a heteroaryl, an alkyl, an alkylene, a cycloalkyl, a hydroxyl, an amino or H, and is preferably the hydroxyl when selected from the hydroxyl, the amino or H; R3 is selected from an aryl, a heteroaryl, an alkyl, an alkylene, a cycloalkyl, a hydroxyl, an amino or H, and is preferably the hydroxyl when selected from the hydroxyl, the amino or H; or, the R1 can form a ring or a heterocycle with the R2 or the R3, the number of C atoms of the ring or the heterocycle is selected from 3-6; the aryl, the heteroaryl, the alkyl, the alkylene, the cycloalkyl or the ring is each independently unsubstituted or substituted, and when substituted, the substituents are each independently selected from a methyl, a methylene, a methoxy, an amino or halo, are preferably halo, and are more preferably F; and the heteroaryl or the heterocycle is each independently unsubstituted or substituted, wherein the heteroatoms are each independently selected from N, O or S, and are preferably N.

Further, the described amide substrate is selected from 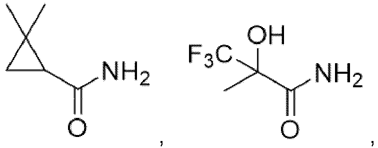 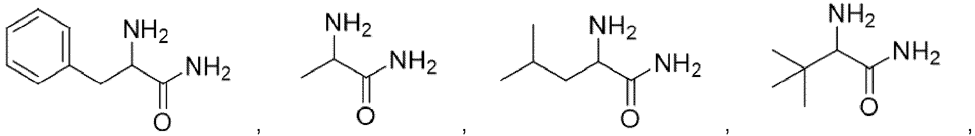 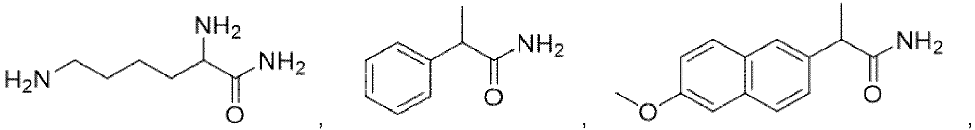 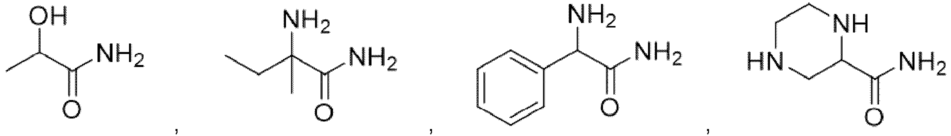 or

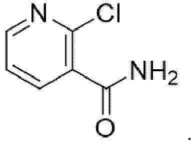

By applying the technical solution of the present application, an amidase mutant derived from *Yersinia frederiksenii* (SEQ ID NO: 1) is used as the parent, and protein engineering modifications such as saturation mutation and combination mutation are carried out to obtain an amidase mutant with greatly improved catalytic activity and stereoselectivity in the synthesis of chiral carboxylic acids and amide derivatives, and the amidase mutant is more suitable for industrial production.

### Brief Description of the Drawings

The accompanying drawings, which form a part of the present application, are used to provide a further understanding of the present application. The schematic embodiments of the present application and the description thereof are used to explain the present application, and do not form improper limits to the present application. In the drawings:

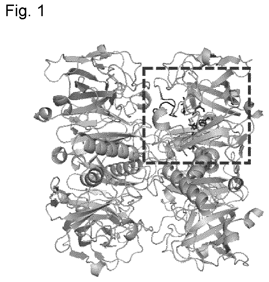

Fig. 1 shows a schematic diagram of the original amidase protein structure for protein engineering modifications of the present application.

### Detailed Description of the Embodiments

It is important to note that the embodiments of the present application and the characteristics in the embodiments can be combined under the condition of no conflicts. Hereinafter, the present application will be described in detail with reference to embodiments.

As mentioned in the background, chiral carboxylic acids and amide derivatives are key intermediates for the synthesis of a large number of chiral bioactive molecules. However, there are many limitations in synthesizing chiral carboxylic acids and amide derivatives by chemical methods. The synthesis of chiral carboxylic acids and chiral amide derivatives by biological amidases has certain advantages over chemical methods. However, wild-type amidases still have certain limitations, deficiencies in catalytic activity, stereoselectivity and stability limit their application in industrial production.

Therefore, in the present application, the inventors try to modify an amidase by using an enzyme evolution method, and then improve the catalytic properties of the amidase, so that the production efficiency can be improved in industrial production, and therefore a series of protection solutions of the present application are proposed.

In a first typical embodiment of the present application, provided is an amidase mutant, comprising: (a) a protein having an amino acid sequence as shown in SEQ ID NO: 1; or (b) a protein having amidase functions after amino acid mutation at least one of the following sites of the amino acid sequence as shown in (a): L81, E62, M311, N180, P121, R58, E32, T230, N82, N190, D192, D217, H219, K77, M78, P79, F80, F61, D185, D59, L182, T183, P184, I227, C228, G229, A231 or V232; or (c) a protein having 80% or more identity with the amino acid sequence defined in any one of (a) and (b) and having amidase functions.

The described amino acid sequence as shown in SEQ ID NO: 1 is an amidase derived from *Yersinia frederiksenii.* By analyzing the protein structure composed of the amino acid sequence (as shown in Fig. 1), 28 sites near the active site and substrate channel are selected for modifications: N82, N190, D192, D217, H219, M311, P121, N180, K77, M78, P79, F80, L81, E32, F61, E62, D185, R58, D59, L182, T183, P184, I227, C228, G229, T230, A231, and V232. These amino acid sites are likely to affect the stereoselectivity of the protein. By mutating the described amino acid sites, a protein having amidase functions or even improved amidase functions can be obtained. The obtained protein can be changed at a non-critical mutation site and active site, and then a protein having 80% or more identity with the described amino acid sequence and having amidase functions can be obtained.

The sequence of SEQ ID NO: 1 is as follows:

MKWLEESIMVKRGVGAGRKPVTHHLTEEMQKEFHYTIGPYSTPVLTIEPGDRVIVDTRDAFEG
AINSEQDIPSQLLKMPFLNPQNGPIMVNGAEKGDVIAVYIESMLPRGVNPHGICAMIPHFGGLTGTD
LTAMLNDPLPEKVRMIKLDSEKVYWSERHTLPYKPHIGTLSVSPEIDSINSLTPDNHGGNMDVPDIG
PGSITYLPVRAPGGRLFIGDAHACQGDGEICGTAVEFASITTIKVDLIKNWQLSWPRMENAETIMSIG
SARPLEDATRIAYRDLIYWLVADFGFDQWDAYMLLSQCGKVRLGNMVDPKYTVGAMLNKELLAP.

In a preferred embodiment, the amino acid mutation of (b) is each independently selected from L81A or L81C or L81V; P79Q; F80G; E62Q or E62K or E62M or E62T or E62A or E62R or E62C or E62V or E62P or E62H or E62N or E62S; L76V; K77T; D185H; R58M or R58K or R58L or R58H or R58A; I227L or I227V or I227A or I227W or I227E or I227S or I227M or I227D or I227C or I227Y; T230C or T230V or T230A or T230L; N180V or N180D or N180K; P121H or P121K or P121N or P121A or P121R or P121E or P121Y or P121S; M311C or M311R or M311H or M311S; V232I; R58L or R58M or R58K or R58Q or R58P or R58A or R58V or R58F; E32D or E32H or E32M or E32Q or E32A or E32C or E32V or E32F or E32I or E32P or E32T; wherein the letter before the number represents the original amino acid, and the letter after the number represents the amino acid after mutation; and preferably, (c) is a protein having 85% or more, preferably 90% or more, more preferably 95% or more, and further preferably 99% or more identity with the amino acid sequence defined in (a) or (b) and having amidase functions.

As used herein, amino acid residues are abbreviated as follows: alanine (Ala; A), asparagine (Asn; N), aspartic acid (Asp; D), arginine (Arg; R), cysteine (Cys; C), glutamic acid (Glu; E), glutamine (Gln; Q), glycine (Gly; G), histidine (His; H), isoleucine (Ile; I), leucine (Leu; L), lysine (Lys; K), methionine (Met; M), phenylalanine (Phe; F), proline (Pro; P), serine (Ser; S), threonine (Thr; T), tryptophan (Trp; W), tyrosine (Tyr; Y) and valine (Val; V).

The rules for substitutions and replacements are that in general, amino acids having similar properties will have similar effects after substitution. For example, in the described homologous proteins, a conservative amino acid replacement may occur. "Conservative amino acid replacement" includes, but is not limited to the following replacements:
a hydrophobic amino acid (Ala, Cys, Gly, Pro, Met, Val, Ile or Leu) is substituted by other hydrophobic amino acid;
a hydrophobic amino acid (Phe, Tyr or Trp) having a large side chain is substituted by other hydrophobic amino acid having a large side chain;
an amino acid (Arg, His or Lys) having a positively charged side chain is replaced by other amino acid having a positively charged chain; and
an amino acid (Ser, Thr, Asn or Gln) having a polar, uncharged side chain is replaced by other amino acid having a polar, uncharged side chain.

A person skilled in the art would also have been able to perform conservative replacements on amino acids according to amino acid replacement rules well known to a person skilled in the art, such as " blosum62 scoring matrix" in the prior art.

In the present application, the applicant continued to explore the described active sites and found that when the active sites were mutated to different amino acids, the corresponding protein activity was also different. Specific mutations can enhance the amidase activity. By means of experimental research, it was found that the described specific mutations at the active sites can obtain proteins having enhanced activity. The amino acid mutation sites of the amidase protein can be flexibly selected and combined in the described mutation.

"Sequence identity" between two polypeptide sequences indicates the percentage of identical amino acids between the sequences, and methods for evaluating the degree of sequence identity between amino acids or nucleotides are known to a person skilled in the art. For example, amino acid sequence identity is typically measured using sequence analysis software. For example, the identity can be determined using the BLAST program of the NCBI database. For determination of sequence identity, see, e.g., Sequence Analysis in Molecular Biology, von Heinje, G., Academic Press, 1987 and Sequence Analysis Primer, Gribskov, M. and Devereux, J., edu., M Stockton Press, New York, 1991.

In a preferred embodiment, the mutation of the amidase mutant comprises any one of the following amino acid mutations:
L81A; L81C; L81V; P79Q; F80G; E62Q; E62K; E62M; E62T; L76V; K77T; D185H; R58M; I227L; I227V; T230C; L81A + E62T; L81A + E62K; L81A + E62M; L81A + I227V; L81A + T230C; L81A+A231R; L81A + L76V; L81A + I227V; L81A + N180V; L81A + N180D; L81A + N180K; L81A + P121H; L81A + P121K; L81A + P121N; L81A + P121A; L81A + M311C; L81A + M311R; L81A + M311H; L81A + M311S; L81A + E62T + P121A; L81A + E62T + P121H; L81A + E62T + P121N; L81A + E62T + P121R; L81A + E62T + I227A; L81A + E62T + M311C; L81A + E62T + M311R; L81A + E62T + M311H; L81A + E62T + M311S; L81A + E62T + T230C; L81A + E62T + T230V; L81A + E62T + V232I; L81A + E62T + R58L; L81A + E62T + R58M; L81A + E62T + R58K; L81A + E62T + R58Q; L81A + E62T + E32D; L81A + E62T + E32H; L81A + E62T + E32M; L81A + E62T + M311C + E32Q; L81A + E62T + M311C + E32M; L81A + E62T + M311C + E32D; L81A + E62T + M311C + E32A; L81A + E62T + M311C + E32C; L81A + E62T + M311C + E32V; L81A + E62T + M311C + P121E; L81A + E62T + M311C + P121A; L81A + E62T + M311C + P121H; L81A + E62T + M311C + P121R; L81A + E62T + M311C + P121N; L81A + E62R + M311C; L81A + E62A + M311C; L81A + E62C + M311C; L81A + E62V + M311C; L81A + E62P + M311C; L81A + E62M + M311C; L81A + E62T + M311C + R58K; L81A + E62T + M311C + R58L; L81A + E62T + M311C + R58H; L81A + E62T + M311C + R58A; L81A + E62T + M311C + I227A; L81A + E62T + M311C + I227L; L81A + E62T + M311C + I227W; L81A + E62T + M311C + I227E; L81A + E62T + M311C + I227S; L81A + E62T + M311C + T230V; L81A + E62T + M311C + T230C; L81A + E62T + M311C + T230A; L81A + E62T + M311C + T230L; L81A + E62A + M311C + T230C; L81A + E62H + M311C + T230C; L81A + E62N + M311C + T230C; L81A + E62R + M311C + T230C; L81A + E62S + M311C + T230C; L81A + E62Q + M311C + T230C; L81A + E62T + M311C + T230C + R58K; L81A + E62T + M311C + T230C + R58P; L81A + E62T + M311C + T230C + R58A; L81A + E62T + M311C + T230C + R58V; L81A + E62T + M311C + T230C + R58F; L81A + E62T + M311C + T230C + R58M; L81A + E62T + M311C + T230C + I227V; L81A + E62T + M311C + T230C + I227M; L81A + E62T + M311C + T230C + I227A; L81A + E62T + M311C + T230C + I227D; L81A + E62T + M311C + T230C + I227C; L81A + E62T + M311C + T230C + I227Y; L81A + E62T + M311C + T230C + P121A; L81A + E62T + M311C + T230C + P121R; L81A + E62T + M311C + T230C + P121E; L81A + E62T + M311C + T230C + P121H; L81A + E62T + M311C + T230C + P121N; L81A + E62T + M311C + T230C + P121Y; L81A + E62A + M311C + T230C + E32F; L81A + E62A + M311C + T230C + E32I; L81A + E62A + M311C + T230C + E32P; L81A + E62A + M311C + T230C + E32T; L81A + E62A + M311C + T230C + I227V; L81A + E62A + M311C + T230C + I227L; L81A + E62A + M311C + T230C + I227A; L81A + E62A + M311C + T230C + T230V; L81A + E62A + M311C + T230C + P121A; L81A + E62A + M311C + T230C + P121R; L81A + E62A + M311C + T230C + P121S.

The described amino acid mutations are all tested and explored in the examples of the present application, and all have amidase activity. Compared with the parent having an amino acid sequence as shown in SEQ ID NO: 1, the described amidase mutants have higher stereoselectivity and catalytic activity, and thus the described amidase mutants can be used for industrial scale-up production.

In a second typical embodiment of the present application, provided is a DNA molecule encoding the described amidase mutant.

In a third typical embodiment of the present application, provided is a recombinant vector, and the recombinant vector is linked with the described DNA molecule.

The described DNA can encode the described amidase mutant, and can be linked to the recombinant vector to form a circular DNA. Both the described DNA and recombinant vector can be transcribed and translated under the action of an RNA polymerase, a ribosome, a tRNA, etc., so as to obtain the described amidase mutant.

In a fourth typical embodiment of the present application, provided is a host cell transformed with the described recombinant vector. The host cell may be a prokaryotic cell or a eukaryotic cell. In particular, the prokaryotic cell may be E. coli, and the eukaryotic cell may be a yeast.

The described host cell can be used to replicate the recombinant vector in the host cell, and can also allow the transcription and translation of the DNA molecule carried on the recombinant vector to obtain a large number of amidase mutants. An existing technology is used to perform protein purification after crushing, crude enzyme catalysis after crushing or other methods on the host cell to obtain an amidase mutant, and the subsequent synthesis reaction of chiral carboxylic acids and chiral amide derivatives can be catalyzed. The host cell is a host cell of a non-plant origin.

In a fifth typical embodiment of the present disclosure, provided is a method for preparing chiral carboxylic acids and amide derivatives. The preparation method comprises using the described amidase mutant to catalyze the hydrolysis resolution reaction of an amide substrate as represented by formula I to obtain chiral carboxylic acids and amide derivatives, wherein R1 is selected from an aryl, a heteroaryl, an alkyl, an alkylene, a cycloalkyl, a hydroxyl, an amino or H, and is preferably the hydroxyl when selected from the hydroxyl, the amino or H; R2 is selected from an aryl, a heteroaryl, an alkyl, an alkylene, a cycloalkyl, a hydroxyl, an amino or H, and is preferably the hydroxyl when selected from the hydroxyl, the amino or H; R3 is selected from an aryl, a heteroaryl, an alkyl, an alkylene, a cycloalkyl, a hydroxyl, an amino or H, and is preferably the hydroxyl when selected from the hydroxyl, the amino or H; or the R1 can form a ring or a heterocycle with the R2 or the R3, the number of C atoms of the ring or the heterocycle is selected from 3-6; the aryl, the heteroaryl, the alkyl, the alkylene, the cycloalkyl or the ring is each independently unsubstituted or substituted, and when substituted, the substituents are each independently selected from a methyl, a methylene, a methoxy, an amino or halo, are preferably halo, and are more preferably F; and the heteroaryl or the heterocycle is each independently unsubstituted or substituted, wherein the heteroatoms are each independently selected from N, O or S, and are preferably N.

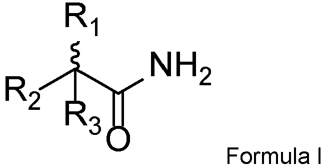

By using the described preparation method, the described amidase mutant is used to catalyze the hydrolysis and resolution reaction of the amide substrate as represented by formula I to obtain chiral carboxylic acids and amide derivatives. The described amidase mutant can be used to perform a hydrolysis and resolution reaction on the described amide substrate. The catalytic activity and the stereoselectivity of the amidase are greatly improved, the production efficiency can be improved, the requirements for industrial production of chiral carboxylic acids and amide derivatives are met, and the industrial production costs are reduced.

In a preferred embodiment, the amide substrate is selected from 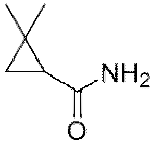 (substrate 1), 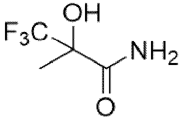 (substrate 2), 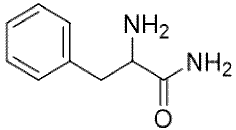 (substrate 3), 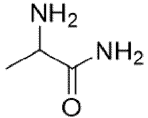 (substrate 4), 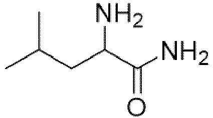 (substrate 5), 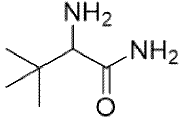 (substrate 6), 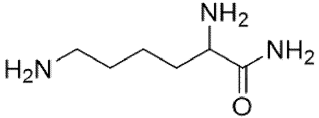 (substrate 7), 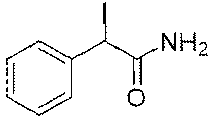 (substrate 8), 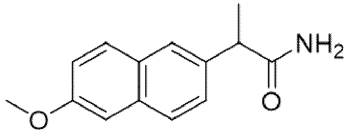 (substrate 9), 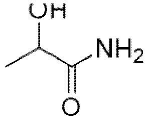 (substrate 10), 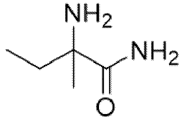 (substrate 11) 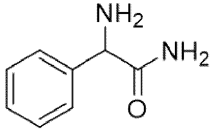 (substrate 12), 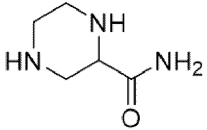 (substrate 13) or 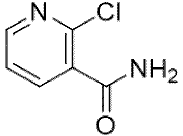 (substrate 14).

In the present application, the inventors selected 28 sites near the active site and substrate channel for modifications on the basis of the analyzed three-dimensional structure (5ubu.1) of the protein: N82, N190, D192, D217, H219, M311, P121, N180, K77, M78, P79, F80, L81, E32, F61, E62, D185, R58, D59, L182, T183, P184, I227, C228, G229, T230, A231, V232. Methods for protein modifications include saturation mutations and combination mutations.

The saturation mutation is a method for obtaining a mutant in which amino acids at target sites are respectively replaced by other 19 amino acids in a short period of time by modifying a gene encoding a protein of interest. This method is not only a powerful tool for targeted modifications of proteins, but also an important means for the study of protein structure-function relationships. Saturation mutation often produces more ideal evolutionary bodies than single point mutation. With regard to problems that cannot be solved by site-specific mutation methods, the saturation mutation method has unique advantages. A saturated mutant is constructed by whole vector PCR, and the PCR product was then digested with DPNI enzyme to remove the template and transformed into Escherichia coli BL21 (DE3). Screening of mutants often requires high throughput screening methods, and therefore we have developed the following method to screen for mutant tolerance.

The following high throughput screening method was developed to screen a mutant library.
1. Mutant cultivation: 300 uL of LB medium was added to each well of a 96-well plate, and single colonies on an agar plate were inoculated into a deep-well 96-well plate and were cultivated overnight at 37°C and 200 rpm; Qpix was used to transfer the overnight culture to another 96-well plate with 800 uL of LB medium added to each well, and the culture was incubated at 37°C and 200 rpm for 5 h; after culturing for 5 h, when the bacterial solution OD600 of the 96-well plate reached 0.6-0.9, Qpix was used again to add an IPTG solution to the 96-well plate, so that the final concentration of IPTG in the well plate was 0.1 mM, and overnight induction was performed at 25°C and 200 rpm for about 16 h; centrifugation was performed for 5 min at 4000 rpm, the supernatant was removed, and a reaction was performed using whole cells.
2. High-throughput screening system for a 96-well plate: 200 ul of a phosphate buffer solution was added to each well of a 96-well plate, the plate was shaken for resuspension, then 2-20 mg of a substrate was added, the plate was shaken at a constant temperature of 30°C, and a reaction was performed at 700 rpm. Different reaction times were controlled according to different screening purposes. After the reaction was finished, 1 ml of acetonitrile was added to the system to stop the reaction, and after centrifugation, the supernatant was taken and sent to UPLC for analysis.

Mutants having improved traits were obtained by the primary mutant screening described above. Then, the optimal mutant was induced and cultivated in a 2 L shake flask (optimal conditions for inducing expression: 25°C, 0.2 mM IPTG for overnight induction). After centrifugation to obtain bacterial sludge, a crude enzyme solution was obtained by ultrasonic cell disruption. Finally, a g-scale reaction was performed to confirm the optimal mutant traits. The described single-point saturation mutations at the described 28 sites often had insignificant improvement effects, so multiple rounds of iterative saturation mutations were generally required to obtain mutants having significantly improved traits.

On the basis of obtaining a mutant having improved activity by means of saturation mutation, beneficial amino acid sites obtained by screening can be combined to obtain a mutant having better traits. The construction method for double-site mutation in combination mutation was the same as that of single-site mutation, and used a whole vector PCR method for construction. A multi-site mutation of two or more sites was performed by overlap extension PCR amplification, and a mutated gene containing multiple mutations was obtained. After restriction endonuclease digestion at both ends, the mutated gene was connected to an expression vector, the expression vector was transformed into *Escherichia coli* cells, and the *Escherichia coli* cells were spread on LB culture dishes containing 100 ug/mL ampicillin. After the described combined mutant was identified as correct by sequencing, it was directly subjected to 2 L shake flask induction culture and reaction verification.

After multiple rounds of evolution, a series of amidase mutants were obtained, and as a result, it was found that the catalytic efficiency of these amidase mutants on various amide compounds was greatly improved. Chiral carboxylic acids and amide derivatives having extremely high chiral purity were obtained, and it was proved that these mutants can be used in the industrial production of chiral carboxylic acids and amide derivatives.

The present application will be further described in detail with reference to the following examples, and these examples should not be construed as limiting the scope of protection of the present application.

### Example 1

The saturation mutation of the described 28 sites was performed on the basis of the parent SEQ ID NO: 1, and the catalytic activity and ee value of the mutant were tested according to the following reaction conditions.

1 mL of reaction system comprised 100 mg of substrate 1 or substrate 2, 10 mg of enzyme powder (obtained by freeze-drying 100 mg of fermented wet bacterial sludge) and 100 mM of a phosphate buffer having pH 7.5. The reaction time was tracked at 30°C. After 2 h of reaction, samples were taken and sent to HPLC to measure the yield. After 16 h, samples were taken and sent to HPLC to measure the ee value.

The detection results are shown in the table below.

**Table 1:**

| Mutation site | Substrate 1 | | Substrate 2 | |
|---|---|---|---|---|
| | Yield | ee value | Yield | ee value |
| parent | - | * | + | * |
| L81A | + | *** | + | *** |
| L81C | + | *** | + | *** |
| L81V | + | ** | + | ** |
| P79Q | + | ** | + | ** |
| F80G | + | ** | + | ** |
| E62Q | ++ | * | +++ | * |
| E62K | ++ | * | +++ | ** |
| E62M | ++ | * | ++ | ** |
| E62T | +++ | ** | ++ | * |
| L76V | + | * | + | ** |
| K77T | + | ** | + | ** |
| D185H | ++ | ** | ++ | * |
| R58M | + | * | + | * |
| I227L | + | * | ++ | ** |
| I227V | ++ | ** | ++ | ** |
| T230C | + | ** | + | ** |

| | | | | |
|---|---|---|---|---|
| Note: In the table above, - represents that the yield of the wild-type parent is 10%, + represents a yield greater than or equal to 20% and less than 30%, ++ represents a yield greater than or equal to 30% and less than 40%, +++ represents a yield greater than or equal to 40% and less than 45%. In addition, in the table above, the ee value is the ee value of R-carboxylic acid when the yield reaches ~50%. * represents an ee value less than 80%, ** represents an ee value greater than or equal to 80% and less than 90%, and *** represents an ee value greater than or equal to 90% and less than 95%. | | | | |

### Example 2

The next round of saturation mutation and combination mutation was performed on the basis of Example 1, and the combined mutant was screened for activity according to the following reaction conditions. The results are shown in the table below.

1 mL of reaction system comprised 100 mg of substrate 1 or substrate 2, 8 mg of enzyme powder (obtained by freeze-drying 80 mg of fermented wet bacterial sludge) and 100 mM of a phosphate buffer having pH 7.5. The reaction time was tracked at 30°C. After 2 h of reaction, samples were taken and sent to HPLC to measure the yield. After 16 h, samples were taken and sent to HPLC to measure the ee value.

**Table 2:**

| Mutation site | Substrate 1 | | Substrate 2 | |
|---|---|---|---|---|
| | Yield | ee value | Yield | ee value |
| L81A | + | *** | + | *** |
| L81A+E62T | +++ | *** | +++ | **** |
| L81A+E62K | +++ | *** | + | *** |
| L81A+E62M | +++ | ** | + | ** |
| L81A+I227V | +++ | ** | + | ** |
| L81A+T230C | ++ | ** | +++ | * |
| L81A+A231R | ++ | ** | +++ | ** |
| L81A+L76V | ++ | * | ++ | ** |
| L81A+I227V | +++ | ** | ++ | * |
| L81A+N180V | ++ | ** | +++ | ** |
| L81A+N180D | ++ | ** | +++ | ** |
| L81A+N180K | ++ | * | +++ | * |
| L81A+P121H | +++ | * | +++ | ** |
| L81A+P121K | +++ | ** | +++ | ** |
| L81A+P121N | +++ | * | +++ | * |
| L81A+P121A | +++ | ** | +++ | ** |
| L81A+M311C | ++ | ** | ++ | ** |
| L81A+M311R | ++ | ** | ++ | * |
| L81A+M311H | ++ | * | ++ | ** |
| L81A+M311S | +++ | ** | ++ | * |

| | | | | |
|---|---|---|---|---|
| Note: In the table above, + represents a yield greater than or equal to 20% and less than 30%, ++ represents a yield greater than or equal to 30% and less than 40%, and +++ represents a yield greater than or equal to 40% and less than 45%. In addition, the ee value in the table above is the ee value of R-carboxylic acid when the yield reaches ~50%. * represents an ee value less than 80%, ** represents an ee value greater than or equal to 80% and less than 90%, *** represents an ee value greater than or equal to 90% and less than 95%, and **** represents an ee value greater than or equal to 95% and less than 98%. | | | | |

### Example 3

Saturation and combination mutations were performed on the basis of Example 2, and the catalytic activity of mutants was detected according to the following reaction conditions.

1 mL of reaction system comprised 100 mg of substrate 1 or substrate 2, 5 mg of enzyme powder (obtained by freeze-drying 50 mg of fermented wet bacterial sludge) and 100 mM of a phosphate buffer having pH 7.5. The reaction time was tracked at 30°C. After 2 h of reaction, samples were taken and sent to HPLC to measure the yield. After 16 h, samples were taken and sent to HPLC to measure the ee value.

**Table 3:**

| Mutation site | Substrate 1 | | Substrate 2 | |
|---|---|---|---|---|
| | Yield | ee value | Yield | ee value |
| L81A+E62T | ++ | *** | ++ | **** |
| L81A+E62T+ P121A | ++ | *** | ++ | **** |
| L81A+E62T+P121H | ++ | *** | +++ | *** |
| L81A+E62T+P121N | +++ | ** | ++ | ** |
| L81A+E62T+P121R | ++ | ** | ++ | ** |
| L81A+E62T+I227A | ++ | ** | ++++ | *** |
| L81A+E62T+M311C | ++++ | **** | +++ | *** |
| L81A+E62T+M311R | ++ | *** | ++ | *** |
| L81A+E62T+M311H | ++ | *** | +++ | *** |
| L81A+E62T+M311S | +++ | *** | ++ | ** |
| L81A+E62T+T230C | +++ | *** | ++ | ** |
| L81A+E62T+T230V | +++ | *** | +++ | *** |
| L81A+E62T+V2321 | ++ | *** | +++ | *** |
| L81A+E62T+R58L | +++ | *** | ++ | **** |
| L81A+E62T+R58M | ++ | *** | +++ | *** |
| L81A+E62T+R58K | +++ | ** | ++ | ** |
| L81A+E62T+R58Q | +++ | ** | ++ | *** |
| L81A+E62T+E32D | ++ | ** | +++ | ** |
| L81A+E62T+E32H | ++ | *** | +++ | *** |
| L81A+E62T+E32M | ++ | *** | +++ | *** |

| | | | | |
|---|---|---|---|---|
| Note: In the table above, + represents a yield greater than or equal to 20% and less than 30%, ++ represents a yield greater than or equal to 30% and less than 40%, and +++ represents a yield greater than or equal to 40% and less than 45%. ++++ represents a yield greater than or equal to 45% and less than 50%. In addition, the ee value in the table above is the ee value of R-carboxylic acid when the yield reaches ~50%. * represents an ee value less than 80%, ** represents an ee value greater than or equal to 80% and less than 90%, *** represents an ee value greater than or equal to 90% and less than 95%, and **** represents an ee value greater than or equal to 95% and less than 98%. | | | | |

### Example 4

Saturation mutation and combination mutation were performed on the basis of Example 3, and the catalytic activity of mutants was detected according to the following reaction conditions.

1 mL of reaction system comprised 100 mg of substrate 1 or substrate 2, 2 mg of enzyme powder (obtained by freeze-drying 20 mg of fermented wet bacterial sludge) and 100 mM of a phosphate buffer having pH 7.5. The reaction time was tracked at 30°C. After 1 h of reaction, samples were taken and sent to HPLC to measure the yield. After 8 h, samples were taken and sent to HPLC to measure the ee value.

**Table 4:**

| Mutation site | Substrate 1 | | Substrate 2 | |
|---|---|---|---|---|
| | Yield | ee value | Yield | ee value |
| L81A+E62T+M311C | ++ | **** | ++ | *** |
| L81A+E62T+M311C+E32Q | ++ | *** | +++ | *** |
| L81A+E62T+M311C+E32M | +++ | *** | ++ | *** |
| L81A+E62T+M311C+E32D | ++ | *** | ++ | *** |
| L81A+E62T+M311C+E32A | ++ | *** | ++++ | *** |
| L81A+E62T+M311C+E32C | ++++ | **** | +++ | *** |
| L81A+E62T+M311C+E32V | ++ | *** | ++ | *** |
| L81A+E62T+M311C+P121E | ++ | *** | +++ | *** |
| L81A+E62T+M311C+P121A | +++ | *** | ++ | *** |
| L81A+E62T+M311C+P121H | +++ | *** | ++ | *** |
| L81A+E62T+M311C+P121R | +++ | *** | +++ | *** |
| L81A+E62T+M311C+P121N | ++ | *** | +++ | *** |
| L81A+E62R+M311C | +++ | *** | ++ | **** |
| L81A+E62A+M311C | ++ | *** | +++ | *** |
| L81A+E62C+M311C | +++ | *** | ++ | *** |
| L81A+E62V+M311C | +++ | *** | ++ | *** |
| L81A+E62P+M311C | ++ | *** | +++ | *** |
| L81A+E62M+M311C | ++ | *** | +++ | *** |
| L81A+E62T+M311C+R58K | ++ | *** | +++ | *** |
| L81A+E62T+M311C+R58L | +++ | *** | ++ | *** |
| L81A+E62T+M311C+R58H | +++ | *** | ++ | *** |
| L81A+E62T+M311C+R58A | ++ | *** | +++ | *** |
| L81A+E62T+M311C+I227A | +++ | ** | ++ | ** |
| L81A+E62T+M311C+I227L | ++ | *** | +++ | *** |
| L81A+E62T+M311C+I227W | +++ | *** | ++ | *** |
| L81A+E62T+M311C+I227E | +++ | *** | ++ | *** |
| L81A+E62T +M311C+I227S | ++ | *** | +++ | *** |
| L81A+E62T+M311C+T230V | ++ | *** | +++ | *** |
| L81A+E62T+M311C+T230C | ++++ | **** | ++++ | **** |
| L81A+E62T+M311C+T230A | ++ | *** | +++ | *** |
| L81A+E62T+M311C+T230L | ++ | *** | +++ | *** |

| | | | | |
|---|---|---|---|---|
| Note: In the table above, + represents a yield greater than or equal to 20% and less than 30%, ++ represents a yield greater than or equal to 30% and less than 40%, and +++ represents a yield greater than or equal to 40% and less than 45%. ++++ represents a yield greater than or equal to 45% and less than 50%. In addition, the ee value in the table above is the ee value of R-carboxylic acid when the yield reaches ~50%. * represents an ee value less than 80%, ** represents an ee value greater than or equal to 80% and less than 90%, *** represents an ee value greater than or equal to 90% and less than 95%, and **** represents an ee value greater than or equal to 95% and less than 98%. | | | | |

### Example 5

Multiple rounds of saturation mutation and combination mutation were performed on the basis of Example 4, and the catalytic activity of the mutants was detected according to the following reaction conditions.

1 mL of reaction system comprised 100 mg of substrate 1 or substrate 2, 1 mg of enzyme powder (obtained by freeze-drying 10 mg of fermented wet bacterial sludge) and 100 mM of a phosphate buffer having pH 7.5. The reaction time was tracked at 30°C. After 1 h of reaction, samples were taken and sent to HPLC to measure the yield. After 8 h, samples were taken and sent to HPLC to measure the ee value.

**Table 5:**

| Mutation site | Substrate 1 | | Substrate 2 | |
|---|---|---|---|---|
| | Yield | ee value | Yield | ee value |
| L81A+E62T+M311C+T230C | +++ | **** | +++ | **** |
| L81A+E62A+M311C+T230C | ++++ | ***** | ++++ | ***** |
| L81A+E62H+M311C+T230C | ++++ | *** | ++++ | *** |
| L81A+E62N+M311C+T230C | ++++ | **** | ++++ | **** |
| L81A+E62R+M311C+T230C | ++++ | **** | ++++ | **** |
| L81A+E62S+M311C+T230C | ++++ | **** | +++ | *** |
| L81A+E62Q+M311C+T230C | ++++ | *** | ++++ | **** |
| L81A+E62T+M311C+T230C+R58K | ++++ | *** | ++++ | **** |
| L81A+E62T+M311C+T230C+R58P | +++ | *** | ++ | *** |
| L81A+E62T+M311C+T230C+R58A | +++ | *** | ++ | **** |
| L81A+E62T+M311C+T230C+R58V | ++++ | *** | +++ | **** |
| L81A+E62T+M311C+T230C+R58F | ++++ | *** | +++ | *** |
| L81A+E62T+M311C+T230C+R58M | +++ | *** | ++ | **** |
| L81A+E62T+M311C+T230C+I227V | ++++ | *** | +++ | *** |
| L81A+E62T+M311C+T230C+I227M | ++++ | **** | ++ | **** |
| L81A+E62T+M311C+T230C+I227A | +++ | **** | ++ | **** |
| L81A+E62T+M311C+T230C+ I227D | ++++ | *** | +++ | *** |
| L81A+E62T+M311C+T230C+I227C | ++++ | *** | +++ | *** |
| L81A+E62T+M311C+T230C+I227Y | +++ | **** | +++ | *** |
| L81A+E62T+M311C+T230C+P121A | ++++ | **** | ++++ | *** |
| L81A+E62T+M311C+T230C+P121R | ++++ | *** | ++++ | *** |
| L81A+E62T+M311C+T230C+P121E | ++++ | *** | +++ | *** |
| L81A+E62T+M311C+T230C+P121H | ++++ | ** | ++++ | **** |
| L81A+E62T+M311C+T230C+P121N | ++++ | *** | ++++ | **** |
| L81A+E62T+M311C+T230C+P121Y | +++ | **** | ++ | *** |
| L81A+E62A+M311C+T230C+E32F | ++++ | **** | ++ | *** |
| L81A+E62A+M311C+T230C+E32I | ++++ | *** | +++ | *** |
| L81A+E62A+M311C+T230C+E32P | +++ | *** | +++ | *** |
| L81A+E62A+M311C+T230C+E32T | ++++ | **** | ++++ | **** |
| L81A+E62A+M311C+T230C+I227V | ++++ | *** | +++ | *** |
| L81A+E62A+M311C+T230C+I227L | +++ | ***** | ++++ | **** |
| L81A+E62A+M311C+T230C+I227A | ++++ | *** | ++++ | *** |
| L81A+E62A+M311C+T230C+T230V | ++++ | ***** | ++++ | ***** |
| L81A+E62A+M311C+T230C+P121A | ++++ | *** | ++++ | *** |
| L81A+E62A+M311C+T230C+P121R | ++++ | **** | ++++ | **** |
| L81A+E62A+M311C+T230C+P121S | +++ | ***** | ++++ | **** |

| | | | | |
|---|---|---|---|---|
| Note: In the table above, + represents a yield greater than or equal to 20% and less than 30%, ++ represents a yield greater than or equal to 30% and less than 40%, and +++ represents a yield greater than or equal to 40% and less than 45%. ++++ represents a yield greater than or equal to 45% and less than 50%. In addition, the ee value in the table above is the ee value of R-carboxylic acid when the yield reaches ~50%. * represents an ee value less than 80%, ** represents an ee value greater than or equal to 80% and less than 90%, *** represents an ee value greater than or equal to 90% and less than 95%, **** represents an ee value greater than or equal to 95% and less than 98%, and ***** represents an ee value greater than or equal to 99%. | | | | |

### Example 6

100 mL of 100 mmol/L phosphate buffer solution and 10 g of 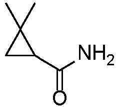 (substrate 1) were added into a 250 mL four-neck flask at room temperature, and stirred until uniform; then 1 mL (prepared from 1 g of wet bacterial sludge) of an enzyme solution of mutant amidase L81A + E62A + M311C + T230C mutated on the basis of SEQ ID NO: 1 was added, and pH was adjusted to 7.5-8.0. The temperature was increased to 30°C, stirring was performed, the reaction time was tracked, and when the yield reached about 50%, the reaction system was stopped. Then post-treatments were respectively performed to finally obtain target products, i.e. an R-carboxylic acid 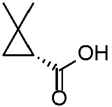 and an S-amide derivative

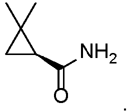

By HPLC detection, the purity of both the R-carboxylic acid and the S-amide derivative was >99%, the ee values were >99%, and the yields were 42% and 40%, respectively.

### Example 7

100 mL of 100 mmol/L phosphate buffer solution and 10 g of 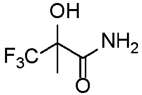 (substrate 2) were added into a 250 mL four-neck flask at room temperature, and stirred until uniform; then 1 mL (prepared from 1 g of wet bacterial sludge) of an enzyme solution of mutant amidase L81A + E62A + M311C + T230C mutated on the basis of SEQ ID NO: 1 was added, and pH was adjusted to 7.5-8.0. The temperature was increased to 30°C, stirring was performed, the reaction time was tracked, and when the yield reached about 50%, the reaction system was stopped. Then post-treatments were respectively performed to finally obtain target products, i.e. an R-carboxylic acid 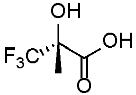 and an S-amide derivative

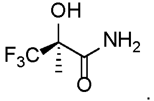

By HPLC detection, the purity of both the R-carboxylic acid and the S-amide derivative was >99%, the ee values were >99%, and the yields were 41% and 43%, respectively.

### Example 8

100 mL of 100 mmol/L phosphate buffer solution and 10 g of 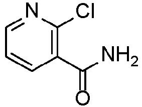 (substrate 14) were added into a 250 mL four-neck flask at room temperature, and stirred until uniform; then 1 mL (prepared from 1 g of wet bacterial sludge) of an enzyme solution of mutant amidase L81A + E62A + M311C + T230C mutated on the basis of SEQ ID NO: 1 was added, and pH was adjusted to 7.5-8.0. The temperature was increased to 30°C, stirring was performed, the reaction time was tracked, and when the yield reached about 95%, the reaction system was stopped. Then post-treatment was performed to finally obtain a target product, i.e. carboxylic acid

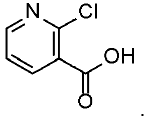

By HPLC detection, the purity of the product carboxylic acid was >99%, the ee value was >99%, and the yield was >84%.

### Example 9

The substrates 3-13 were subjected to a catalytic reaction using an enzyme solution of the amidase mutant L81A + E62A + M311C + T230C mutated on the basis of SEQ ID NO: 1 with reference to the catalytic synthesis steps in Examples 6 to 8. The results are shown in the following table.

**Table 6:**

| Substrate | Product R-carboxylic acid | | | Product S-amide | | |
|---|---|---|---|---|---|---|
| | Purity | ee value | Yield | Purity | ee value | Yield |
| 3 | > 99% | > 99% | 43% | > 99% | > 99% | 42% |
| 4 | > 99% | > 99% | 42% | > 99% | > 99% | 43% |
| 5 | > 98% | > 99% | 41% | > 98% | > 99% | 42% |
| 6 | > 99% | > 99% | 42% | > 99% | > 99% | 38% |
| 7 | > 99% | > 99% | 39% | > 98% | > 99% | 41% |
| 8 | > 98% | > 99% | 42% | > 99% | > 99% | 43% |
| 9 | > 98% | > 99% | 44% | > 99% | > 99% | 42% |
| 10 | > 99% | > 99% | 37% | > 99% | > 99% | 40% |
| 11 | > 98% | > 99% | 40% | > 98% | > 99% | 42% |
| 12 | > 98% | > 99% | 41% | > 98% | > 99% | 39% |
| 13 | > 98% | > 99% | 42% | > 99% | > 99% | 42% |

From the described description, it can be determined that the described examples of the present application achieve the following technical effects: 1) The amidase mutant having improved activity and stereoselectivity can be used to perform an amide resolution reaction to obtain a series of chiral carboxylic acids and amide derivatives having high activity and selectivity. 2) The amidase mutant is used for biocatalysis under mild reaction conditions, without the need for heavy metal catalysts and toxic reagents, thus achieving green chemistry. 3. The amidase has high biocatalytic activity, a high reaction substrate concentration and a high product yield, greatly reduces wastewater, waste gases and waste residues, and saves production costs.

The description above is only the preferred embodiments of the present application, and is not intended to limit the present application. For a person skilled in the art, the present application may have various modifications and variations. Any modifications, equivalent replacements, improvements and the like made within the spirit and principle of the present application shall fall within the scope of protection of the present application.

## Claims

1. An amidase mutant, comprising:
(a) a protein having an amino acid sequence set forth in SEQ ID NO: 1; or
(b) a protein having an amino acid mutation at one or more of the following sites of the amino acid sequence shown in (a): L81, E62, M311, N180, P121, R58, E32, T230, N82, N190, D192, D217, H219, K77, M78, P79, F80, F61, D185, D59, L182, T183, P184, 1227, C228, G229, A231 or V232, and having an amidase function; or
(c) a protein having more than 80% identity with the amino acid sequence defined in any one of (a) and (b) and having an amidase function.

2. The amidase mutant according to claim 1, wherein the amino acid mutation of (b) is each independently selected from the following:
L81A or L81C or L81V;
P79Q;
F80G;
E62Q, E62K, E62T, E62A, E62R, E62C, E62V, E62P, E62H, E62N or E62S;
L76V;
K77T;
D185H;
R58M, R58K, R58L, R58H or R58A;
I227L, I227V, I227A, I227W, I227E, I227S, I227M, I227D, I227C or I227Y;
T230C, T230V, T230A or T230L;
N180V, N180D or N180K;
P121H, P121K, P121N, P121A, P121R, P121E, P121Y or P121S;
M311C, M311R, M311H or M311S;
V232I;
R58L, R58M, R58K, R58Q, R58P, R58A, R58V or R58F; and
E32D, E32H, E32M, E32Q, E32A, E32C, E32V, E32F, E32I, E32P or E32T;
wherein the letter before the number represents the original amino acid, and the letter after the number represents the mutated amino acid;
preferably, the protein in the (c) is a protein having more than 85%, preferably more than 90%, more preferably more than 95%, and further more preferably more than 99% identity with the amino acid sequence defined in (a) or (b) and having an amidase function.

3. The amidase mutant according to claim 2, wherein the amino acid mutation of the amidase mutant comprises any one of the following:
L81A;
L81C;
L81V;
P79Q;
F80G;
E62Q;
E62K;
E62M;
E62T;
L76V;
K77T;
D185H;
R58M;
I227L;
I227V;
T230C;
L81A+E62T;
L81A+E62K;
L81A+E62M;
L81A+I227V;
L81A+T230C;
L81A+A231R;
L81A+L76V;
L81A+1227V;
L81A+N180V;
L81A+N180D;
L81A+N180K;
L81A+P121H;
L81A+P121K;
L81A+P121N;
L81A+P121A;
L81A+M311C;
L81A+M311R;
L81A+M311H;
L81A+M311S;
L81A+E62T+ P121A;
L81A+E62T+P121H;
L81A+E62T+P121N;
L81A+E62T+P121R;
L81A+E62T+I227A;
L81A+E62T+M311C;
L81A+E62T+M311R;
L81A+E62T+M311H;
L81A+E62T+M311S;
L81A+E62T+T230C;
L81A+E62T+T230V;
L81A+E62T+V232I;
L81A+E62T+R58L;
L81A+E62T+R58M;
L81A+E62T+R58K;
L81A+E62T+R58Q;
L81A+E62T+E32D;
L81A+E62T+E32H;
L81A+E62T+E32M;
L81A+E62T+M311C+E32Q;
L81A+E62T+M311C+E32M;
L81A+E62T+M311C+E32D;
L81A+E62T+M311C+E32A;
L81A+E62T +M311C+E32C;
L81A+E62T +M311C+E32V;
L81A+E62T+M311C+P121E;
L81A+E62T+M311C+P121A;
L81A+E62T+M311C+P121H;
L81A+E62T+M311C+P121R;
L81A+E62T+M311C+P121N;
L81A+E62R+M311C;
L81A+E62A+M311C;
L81A+E62C+M311C;
L81A+E62V+M311C;
L81A+E62P+M311C;
L81A+E62M+M311C;
L81A+E62T+M311C+R58K;
L81A+E62T+M311C+R58L;
L81A+E62T+M311C+R58H;
L81A+E62T+M311C+R58A;
L81A+E62T+M311C+I227A;
L81A+E62T+M311C+I227L;
L81A+E62T+M311C+I227W;
L81A+E62T+M311C+I227E;
L81A+E62T+M311C+I227S;
L81A+E62T+M311C+T230V;
L81A+E62T+M311C+T230C;
L81A+E62T+M311C+T230A;
L81A+E62T+M311C+T230L;
L81A+E62A+M311C+T230C;
L81A+E62H+M311C+T230C;
L81A+E62N+M311C+T230C;
L81A+E62R+M311C+T230C;
L81A+E62S+M311C+T230C;
L81A+E62Q+M311C+T230C;
L81A+E62T+M311C+T230C+R58K;
L81A+E62T+M311C+T230C+R58P;
L81A+E62T+M311C+T230C+R58A;
L81A+E62T +M311C+ T230C+R58V;
L81A+E62T+M311C+T230C+R58F;
L81A+E62T+M311C+T230C+R58M;
L81A+E62T+M311C+T230C+I227V;
L81A+E62T+M311C+T230C+I227M;
L81A+E62T+M311C+T230C+I227A;
L81A+E62T+M311C+T230C+I227D;
L81A+E62T+M311C+T230C+I227C;
L81A+E62T+M311C+T230C+I227Y;
L81A+E62T+M311C+T230C+P121A;
L81A+E62T+M311C+T230C+P121R;
L81A+E62T+M311C+T230C+P121E;
L81A+E62T+M311C+T230C+P121H;
L81A+E62T+M311C+T230C+P121N;
L81A+E62T+M311C+T230C+P121Y;
L81A+E62A+M311C+T230C+E32F;
L81A+E62A+M311C+T230C+E32I;
L81A+E62A+M311C+T230C+E32P;
L81A+E62A+M311C+T230C+E32T;
L81A+E62A+M311C+T230C+I227V;
L81A+E62A+M311C+T230C+I227L;
L81A+E62A+M311C+T230C+1227A;
L81A+E62A+M311C+T230C+T230V;
L81A+E62A+M311C+T230C+P121A;
L81A+E62A+M311C+T230C+P121R or
L81A+E62A+M311C+T230C+P121S.

4. A DNA molecule encoding the amidase mutant according to any one of claims 1 to 3.

5. A recombinant vector connected with the DNA molecule of claim 4.

6. A host cell transformed with the recombinant vector of claim 5.

7. A method for preparing a chiral carboxylic acid and a chiral amide derivative, comprising applying the amidase mutant according to any one of claims 1 to 3 to catalyze a hydrolysis resolution reaction of an amide substrate as shown in a formula I, to obtain the chiral carboxylic acid and the chiral amide derivative, 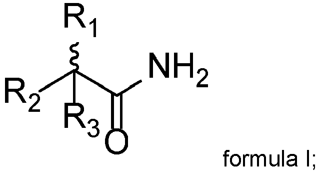
R1 is selected from an aryl group, a heteroaryl group, an alkyl group, an alkylene group, a cycloalkyl group, a hydroxyl group, an amino group or H, and when R1 is selected from the hydroxyl group, the amino group or H, R1 is preferably the hydroxyl group;
R2 is selected from an aryl group, a heteroaryl group, an alkyl group, an alkylene group, a cycloalkyl group, a hydroxyl group, an amino group or H, and when R2 is selected from the hydroxyl group, the amino group or H, R2 is preferably the hydroxyl group;
R3 is selected from an aryl group, a heteroaryl group, an alkyl group, an alkylene group, a cycloalkyl group, a hydroxyl group, an amino group or H, and when R3 is selected from the hydroxyl group, the amino group or H, R3 is preferably the hydroxyl group;
or, the R1 can form a ring or a heterocyclic ring with the R2 or the R3, and the number of C atoms of the ring or the heterocyclc ring is selected from 3 to 6;
the aryl group, the heteroaryl group, the alkyl group, the alkylene group, the cycloalkyl group or the ring is each independently unsubstituted or substituted, when substituted, the substituents are each independently selected from a methyl group, a methylene group, a methoxy group, an amino group or halo, are preferably halo, and are more preferably F;
the heteroaryl group or the heterocyclic ring is each independently unsubstituted or substituted, wherein the heteroatoms are each independently selected from N, O or S, are preferably N.

8. The method according to claim 7, wherein the amide substrate is selected from:
